# EUROPÄISCHE PATENTSCHRIFT

(11) **EP 3 456 703 B1**
(45) Veröffentlichungstag und Bekanntmachung des Hinweises auf die Patenterteilung: **29.04.2020**
(21) Anmeldenummer: 17190901.3
(22) Anmeldetag: 13.09.2017
(51) Int. Cl.: C07C 5/48, C07C 7/00, C07C 7/04, C07C 7/09, C07C 7/12, C07C 11/04

(54) **VERFAHREN ZUR HERSTELLUNG VON ETHYLEN**
METHOD FOR PRODUCING ETHYLENE
PROCÉDÉ DE FABRICATION D'ÉTHYLÈNE

(43) Veröffentlichungstag der Anmeldung: 20.03.2019
(73) Patentinhaber: Linde AG, 80331 München (DE)
(72) Erfinder: FRITZ, Helmut, 81375 München (DE); OBERMEIER, Andreas, 85658 Egmating (DE); PESCHEL, Andreas, 82515 Wolfratshausen (DE); DUC, Tuat Pham, 82377 Penzberg (DE); TOTA, Desislava, 81479 München (DE)
(74) Vertreter: Frommberger, Moritz

(56) Entgegenhaltungen:
- EP-A1- 0 816 290
- EP-A1- 1 024 187
- EP-A1- 3 278 860
- DD-A1- 275 452
- DE-A1- 3 813 976
- DE-A1-102008 060 783
- US-A1- 2017 113 983

## Beschreibung

Die Erfindung betrifft ein Verfahren zur Herstellung von Ethylen.

### Stand der Technik

Die oxidative Dehydrierung (engl. Oxidative Dehydrogenation, ODH) von Paraffinen mit zwei bis vier Kohlenstoffatomen ist grundsätzlich bekannt. Bei der ODH werden die genannten Paraffine mit Sauerstoff unter anderem zu den Olefinen gleicher Kohlenstoffzahl und zu Wasser umgesetzt.

Die ODH kann gegenüber etablierteren Verfahren zur Herstellung von Olefinen wie dem Steamcracken oder der katalytischen Dehydrierung vorteilhaft sein. So besteht aufgrund der Exothermie der beteiligten Reaktionen keine thermodynamische Gleichgewichtslimitierung. Die ODH kann bei vergleichsweise geringen Reaktionstemperaturen durchgeführt werden. Grundsätzlich ist keine Regenerierung der eingesetzten Katalysatoren erforderlich, da die Anwesenheit von Sauerstoff eine insitu-Regenerierung ermöglicht. Schließlich werden im Gegensatz zum Steamcracken geringere Mengen an wertlosen Nebenprodukten wie Koks gebildet.

Zu weiteren Details bezüglich der ODH sei auf einschlägige Fachliteratur, beispielsweise Ivars, F. und Lopez Nieto, J. M., Light Alkanes Oxidation: Targets Reached and Current Challenges, in: Duprez, D. und Cavani, F. (Hrsg.), Handbook of Advanced Methods and Processes in Oxidation Catalysis: From Laboratory to Industry, London 2014: Imperial College Press, Seiten 767 bis 834, oder Gärtner, C.A. et al., Oxidative Dehydrogenation of Ethane: Common Principles and Mechanistic Aspects, ChemCatChem, Bd. 5, Nr. 11, 2013, Seiten 3196 bis 3217, verwiesen.

Gemäß der DE 10 2008 060 783 A1 wird Luft durch Vakuum-Druckwechseladsorption bearbeitet. Ein erhaltener sauerstoffreicher Strom wird durch Tieftemperaturrektifikation in einen Sauerstoffstrom und in einen Argonproduktstrom getrennt.

Die DE 38 13 976 A1 betrifft ein Verfahren zur Gewinnung eines heliumreichen Helium-Wasserstoff-Gemisches aus einem Abblasegas einer Ammoniaksyntheseanlage, wobei das Abblasegas durch partielle Kondensation, Druckwechseladsorption und Tieftemperaturrektifikation von enthaltenem Methan, Argon und Stickstoff befreit wird.

In der EP 3 278 860 A1 werden ein Prozess und eine Anlage zur Erzeugung und Verarbeitung von Produkten der oxidativen Kopplung von Methan vorgeschlagen, bei dem aus einem Produktgemisch mittels Vakuum-Druckwechseladsorption Methan und niedriger als Methan siedende Komponenten entfernt werden.

Die EP 0 816 290 A1 offenbart ein Verfahren zur gleichzeitigen Rückgewinnung von reinem Kohlenmonoxid und reinem Wasserstoff aus einem Reformiergas.

In der EP 1 034 187 A1 wird ein Verfahren zur Rückgewinnung einer ethylenreichen Fraktion aus einer olefinhaltigen Mischfraktion, die aus einem Crackofen entnommen wird, vorgeschlagen. Es erfolgt eine kombinierte Adsorption und Rektifikation.

Die US 2017/113983 A1 bezieht sich auf ein Verfahren zur oxidativen Dehydrierung von Ethan zu Ethylen. Ethylen und gegebenenfalls Ethan in einem Produktgas werden an dinem Sorptionsmittel sorbiert.

Die DD 275 452 A1 betrifft ein Verfahren zur Herstellung von Ethen durch oxidative Dehydrodimerisierung von Methan mit Sauerstoff. In einem Kreislaufprozeß wird an einem Mischoxidkatalysator ein zurückgeführtes Methan-Ethan-Gemisch umgesetzt. Wasser und Kohlendioxid werden aus einem Reaktionsgasgemisch abgetrennt und aus dem wasser- und kohlendioxidfreien Gasgemisch wird Ethen durch kombinierte Druckwechsel-Temperaturwechseladsorptions abgetrennt.

Die Erfindung wird nachfolgend insbesondere unter Bezugnahme auf die ODH von Ethan (sogenannte ODH-E) beschrieben. Neben der oxidativen Dehydrierung von Ethan kann aber prinzipiell auch eine nichtoxidative Dehydrierung von Ethan zur Herstellung von Ethylen erfolgen. Auch für ein derartiges Verfahren eignet sich die vorliegende Erfindung.

Neben den Hauptprodukten Ethylen und Wasser werden bei höheren Umsätzen in der ODH, insbesondere der ODH-E, nennenswerte Mengen an Kohlenmonoxid und Kohlendioxid und ggf. Essigsäure als Nebenprodukte gebildet. Abhängig von den Reaktionsbedingungen kann auch Restsauerstoff in einem entsprechenden Prozessgas, d.h. einem dem Reaktor entnommenen Gasgemisch, enthalten sein. Methan kann ebenfalls als Nebenprodukt gebildet werden oder bereits im Einsatz in den Reaktor enthalten sein und den Reaktor als sich inert verhaltende Komponente im Wesentlichen unbeeinflusst durchlaufen. Die genannten Komponenten, müssen in nachgeschalteten Trennschritten aus dem Prozessgas abgetrennt werden.

Wie auch nachfolgend erläutert, ist es insbesondere aufgrund der geringeren Methangehalte in einem Prozessgas der ODH-E, falls dieses überhaupt enthalten ist, nicht ohne weiteres möglich, bekannte Trennverfahren und Trenneinrichtungen, wie sie beispielsweise für die Trennung von Prozessgasen aus Steamcrackern verwendet werden, für entsprechende Prozessgase aus der ODH-E einzusetzen, ohne Produkt- und Eduktverluste in Kauf zu nehmen. Dies betrifft insbesondere einen Trennschritt, in dem Ethan und Ethylen und höher siedende Komponenten, falls in dem Prozessgas vorhanden, von tiefer siedenden Komponenten abgetrennt werden. Dieser Schritt entspricht grundsätzlich einer sogenannten Demethanisierung in einer Trennung eines Prozessgases aus einem Steamcracker.

Die vorliegende Erfindung stellt sich die Aufgabe, entsprechende Verfahren zu verbessern und die genannten Probleme in einer entsprechenden Trennung, insbesondere für ein Prozessgas aus der ODH-E, zu adressieren.

### Offenbarung der Erfindung

Vor diesem Hintergrund schlägt die vorliegende Erfindung ein Verfahren zur Herstellung von Ethylen, insbesondere durch die erläuterte oxidative Dehydrierung von Ethan vor. Ausgestaltungen sind jeweils Gegenstand der abhängigen Patentansprüche sowie der nachfolgenden Beschreibung.

Stoffströme, Gasgemische und dergleichen können im hier verwendeten Sprachgebrauch "überwiegend" eine oder mehrere Komponenten enthalten, wobei diese Angabe für einen Gehalt von wenigstens 90%, 95%, 99%, 99,5%, 99,9% oder 99,99% auf molarer, Gewichts- oder Volumenbasis stehen kann. Sind mehrere Komponenten angegeben, bezieht sich die Angabe "überwiegend" auf die Summe aller Komponenten. Ist hier beispielsweise von "Sauerstoff", "Methan" oder "Ethylen" die Rede, kann es sich um ein Reingas, aber auch Gemisch handeln, das überwiegend diese jeweiligen Komponenten enthält.

Stoffströme, Gasgemische usw. können im hier verwendeten Sprachgebrauch außerdem "angereichert" oder "abgereichert" an einer oder mehreren Komponenten sein, wobei sich diese Begriffe auf einen Gehalt in einem Ausgangsgemisch beziehen. Sie sind "angereichert", wenn sie zumindest den 1,5-fachen, 2-fachen, 5-fachen, 10-fachen, 100-fachen oder 1.000-fachen Gehalt, "abgereichert", wenn sie höchstens den 0,75-fachen, 0,5-fachen, 0,1-fachen, 0,01-fachen oder 0,001-fachen Gehalt einer oder mehrerer Komponenten, bezogen auf das Ausgangsgemisch, enthalten.

Nachfolgend werden zur Charakterisierung von Drücken und Temperaturen die Begriffe "Druckniveau" und "Temperaturniveau" verwendet, wodurch zum Ausdruck gebracht werden soll, dass Drücke und Temperaturen nicht in Form exakter Druck- bzw. Temperaturwerte vorliegen werden müssen. Ein Druckniveau oder Temperaturniveau kann beispielsweise ± 1%, 5%, 10% oder 20% um einen Mittelwert liegen. Mehrere Druck- und Temperaturniveaus können disjunkte oder überlappende Bereiche darstellen. Dasselbe Druck- bzw. Temperaturniveau kann beispielsweise auch dann noch vorliegen, wenn sich aufgrund von Leitungsverlusten oder Abkühlung Drücke und Temperaturen reduziert haben. Bei hier in bar angegebenen Druckniveaus handelt es sich um Absolutdrücke.

Bei einer "Rektifikationskolonne" handelt es sich im hier verwendeten Sprachgebrauch um eine Trenneinheit, die dafür eingerichtet ist, ein gasförmig oder flüssig oder in Form eines Zweiphasengemischs mit flüssigen und gasförmigen Anteilen, ggf. auch im überkritischen Zustand, eingespeistes Stoffgemisch durch Rektifikation zumindest teilweise aufzutrennen, also aus dem Stoffgemisch jeweils Reinstoffe oder zumindest Stoffgemische mit anderer Zusammensetzung zu erzeugen. Typischerweise sind Rektifikationskolonnen als zylindrische Metallbehälter ausgebildet, die mit Einbauten, beispielsweise Trennböden oder geordneten oder ungeordneten Packungen, ausgerüstet sind. Eine Rektifikationskolonne weist einen Sumpfverdampfer auf. Hierbei handelt es sich um eine Einrichtung mit einem Wärmetauscher, der beheizt wird und dafür eingerichtet ist, eine im Sumpf der Rektifikationskolonne anfallende flüssige Fraktion, auch als Sumpfflüssigkeit bezeichnet, zu erwärmen. Mittels eines Sumpfverdampfers wird kontinuierlich ein Teil des Sumpfprodukts verdampft und gasförmig in die Rektifikationskolonne zurückgespeist.

Die vorliegende Erfindung betrifft Trennverfahren, die dem grundlegenden Konzept nach, nicht jedoch in der erfindungsgemäß realisierten Ausführung, Trennverfahren und Trenneinrichtungen ähneln können, wie sie für andere Prozessgase bekannt sind, beispielsweise aus Steamcrackern. Entsprechende Trennverfahren und Trenneinrichtungen sind beispielsweise im Artikel "Ethylene" in Ullmann's Encyclopedia of Industrial Chemistry, Onlineausgabe, 15. April 2007, DOI 10.1002/14356007.a10_045.pub2, beschrieben. Ein wesentlicher Schritt in derartigen Trennverfahren ist häufig die sogenannte Demethanisierung, in der aus dem Prozessgas, ggf. bereits nach Abtrennung weiterer Komponenten, Methan und tiefer als Methan siedende Verbindungen von höher siedenden Komponenten abgetrennt werden. Zu Details bekannter Demethanisierungsverfahren sei auf die zitierte Fachliteratur verwiesen.

### Vorteile der Erfindung

Wie eingangs erwähnt, sind in einem typischen, mittels ODH-E gebildeten Prozessgas neben Hauptprodukten wie Ethylen (und ggf. Essigsäure) unter anderem nicht umgesetztes Ethan, Kohlenmonoxid und Kohlendioxid sowie ggf. Sauerstoff und Methan enthalten. Das Prozessgas enthält typischerweise auch Wasser sowie ggf. geringere Mengen Inertgase, wobei unter "Inertgasen" hier allgemein solche Gase verstanden werden, die in der ODH nicht oder allenfalls zu geringen Anteilen reagieren, und nicht nur die klassischen Inertgase wie Stickstoff oder Edelgase. Auch Methan verhält sich in der ODH-E im Wesentlichen inert.

Entsprechendes gilt in vergleichbarer Weise für Prozessgase aus anderen Verfahren zur Herstellung von Ethylen, beispielsweise der (nichtoxidativen) Dehydrierung von Ethan, wobei nachfolgend, wie mehrfach erwähnt, vereinfachend auf die ODH-E Bezug genommen wird. In einer der nachgeschalteten Trennung müssen die genannten Nebenprodukte von dem oder den gewünschten Hauptprodukten sowie dem nicht umgesetzten Ethan und den Inertgasen abgetrennt werden.

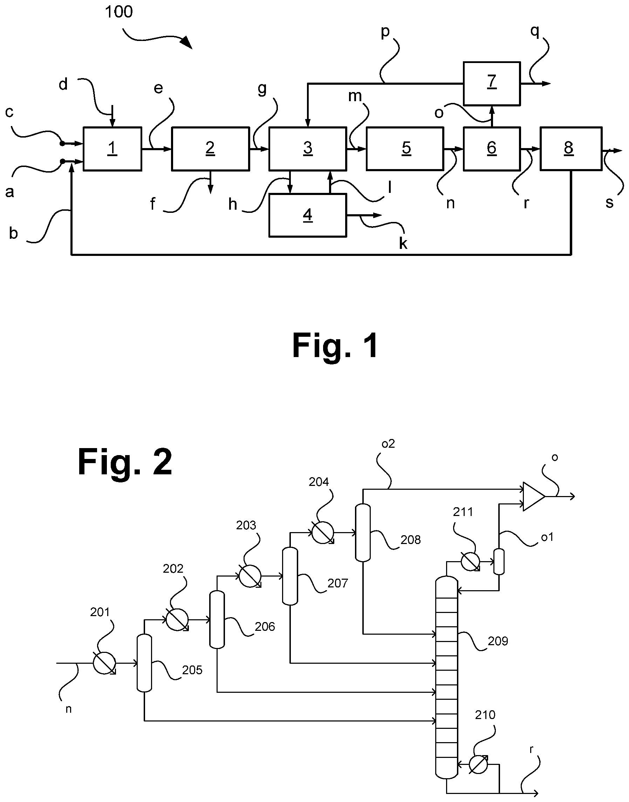

Die Trennung erfolgt, wie auch unter Bezugnahme auf die beigefügte Figur 1 veranschaulicht, typischerweise nach einer Abkühlung und Kondensation des Prozessgases, einer Entfernung von Kohlendioxid, einer Verdichtung und einer Trocknung. Das nach den genannten Schritten noch im Wesentlichen Ethylen, nicht umgesetztes Ethan, Sauerstoff, Kohlenmonoxid, ggf. Methan, sowie geringere Mengen anderer Komponenten enthaltende Prozessgas wird einer Tieftemperaturtrennung unterworfen, in der beispielsweise eine stufenweise Kondensation des Prozessgases erfolgt. Die verbleibenden Gasfraktionen werden jeweils dem nächsten Kondensationsschritt zugeführt. Herkömmlicherweise werden die Kondensate einer Tieftemperaturrektifikation unterworfen, wobei eine Gasfraktion sowie eine flüssige Fraktion gebildet werden. Die Gasfraktion aus der Tieftemperaturrektifikation wird herkömmlicherweise mit der stromab des letzten Kondensationsschritts verbleibenden Gasfraktion zu einer weiteren Gasfraktion, der sogenannten Brenngasfraktion bzw. Tailgasfraktion, vereinigt und einer thermischen Nutzung zugeführt. Die flüssige Fraktion der Tieftemperaturrektifikation wird weiteren Trennschritten unterworfen.

Die Tailgasfraktion soll dabei zumindest den überwiegenden Teil des in dem Prozessgas, das der Tieftemperaturtrennung zugeführt wurde, enthaltenen Sauerstoffs, Kohlenmonoxids und Methans, soweit vorhanden, enthalten. Ethylen und Ethan sollen hingegen zumindest überwiegend in die flüssige Fraktion aus der Tieftemperaturrektifikation übergehen, gemeinsam mit höher siedenden Verbindungen, falls in dem Prozessgas vorhanden und nicht bereits zuvor abgetrennt. Grundsätzlich sollen Produktverluste durch den Übergang entsprechender Verbindungen in die Tailgasfraktion vermieden werden.

Die Trenneffizienz in einer klassischen Tieftemperaturrektifikation, wie sie beispielsweise zur Demethanisierung bei Dampfspaltverfahren eingesetzt wird, richtet sich jedoch wesentlich nach dem Gehalt an Methan in dem Prozessgas, das der Tieftemperaturrektifikation zugeführt wird, weil hierbei ein flüssiger Rücklauf verwendet wird, der im Wesentlichen aus Methan gebildet wird. Bei zu geringen Gehalten an Methan kann der Rücklauf nicht oder nicht in ausreichender Menge bereitgestellt werden. Daher ergeben sich bei zu geringen Mengen an Methan zu hohe Verluste an Ethylen und nicht umgesetztem Ethan in die Tailgasfraktion, und damit wirtschaftliche Nachteile gegenüber anderen Verfahren.

Die vorliegende Erfindung löst diese Probleme in einem Verfahren zur Herstellung von Ethylen, bei dem unter Einsatz einer Dehydrierung von Ethan ein Prozessgas gebildet wird, das zumindest Ethan, Ethylen und tiefer als Ethan und Ethylen siedende Verbindungen enthält, und bei dem unter Verwendung zumindest eines Teils des Prozessgases ein Trenneinsatz gebildet wird, der einer Tieftemperaturtrennung unterworfen wird, in welcher der Trenneinsatz abgekühlt und ein oder mehrere Kondensate aus dem Trenneinsatz abgeschieden werden. Zumindest das oder die Kondensate, in Ausführungsformen jedoch auch ein bei einer entsprechenden Kondensation gasförmig verbleibender Rest, werden zumindest zum Teil unter Erhalt einer gasförmigen ersten Fraktion und einer flüssigen zweiten Fraktion einer Tieftemperaturrektifikation unterworfen, wobei die gasförmige erste Fraktion zumindest das Ethan und das Ethylen in einem geringeren Anteil als in dem Trenneinsatz und die tiefer als Ethan und Ethylen siedenden Verbindungen in einem höheren Anteil als in dem Trenneinsatz enthält.

Die gasförmige erste Fraktion enthält zwar zumindest das Ethan und Ethylen in einem geringeren Anteil als in dem Trenneinsatz, dennoch sind noch, je nach Ausführungsform, mehr oder weniger große Mengen davon in der gasförmigen ersten Fraktion vorhanden. Daher sieht die Erfindung vor, dass die gasförmige erste Fraktion zumindest zum Teil einer Druckwechseladsorption unterworfen wird, mittels welcher eine überwiegend oder ausschließlich Ethan und Ethylen enthaltende dritte Fraktion und eine überwiegend oder ausschließlich die tiefer als Ethan und Ethylen siedenden Verbindungen enthaltende vierte Fraktion gebildet werden. Durch den Einsatz der Druckwechseladsorption können Ethan und Ethylen auf einfache und effiziente Weise zurückgewonnen und dem Verfahren erneut zugeführt werden. Dies hat eine Steigerung der Gesamteffizienz des Verfahrens zur Folge, da das zurückgewonnene Ethan und Ethylen stofflich und nicht thermisch genutzt werden können.

Die Grundidee der vorliegenden Erfindung besteht also in einer Kombination einer Tieftemperaturtrennung mit einer Druckwechseladsorption. Hierbei können unterschiedliche Ausführungsformen vorgesehen sein, in denen jeweils eine Kühlung des Trenneinsatzes in der Tieftemperaturtrennung auf ein bestimmtes Temperaturniveau vorgenommen wird, so dass sich ein oder mehrere Kondensate abscheiden. Diese Kondensate, in bestimmten Ausführungsformen wie erwähnt jedoch auch ein gasförmig verbleibender Rest, werden zumindest zum Teil der Tieftemperaturrektifikation unterworfen. Die Ausführungsformen der vorliegenden Erfindung unterscheiden sich insbesondere, aber nicht nur, durch die Temperaturniveaus, auf die die Abkühlung des Trenneinsatzes zum Zwecke der Abscheidung des oder der Kondensate erfolgt, und ferner durch die Frage, ob eine Einspeisung des gasförmigen Rests in die Tieftemperaturrektifikation vorgenommen wird oder nicht. Weitere Details der genannten und weiterer Ausführungsformen werden nachfolgend erläutert. Eine Druckwechseladsorption ist in jedem Fall Teil der erläuterten Ausführungsformen.

Grundsätzlich kann ein im Rahmen der vorliegenden Erfindung behandeltes Prozessgas direkt am Austritt des oder der verwendeten Reaktoren beispielsweise bis 40 Molprozent Ethan, 5 bis 40 Molprozent Ethylen, 0 bis 10 Molprozent Essigsäure, 0 bis 3 Molprozent Kohlendioxid, 0 bis 5 Molprozent Kohlenmonoxid, 5 bis 70 Molprozent Wasser, 0 bis 5 Molprozent Sauerstoff, 0 bis 5 Molprozent Methan und 0 bis 50 Molprozent Stickstoff enthalten. Weitere Komponenten können insgesamt in einem Gehalt von 0 bis 3 Molprozent enthalten sein. Stromauf der erfindungsgemäß vorgeschlagenen Schritte werden insbesondere Essigsäure, Wasser und Kohlendioxid entfernt, so dass der im Rahmen der vorliegenden Erfindung eingesetzte Trenneinsatz überwiegend oder ausschließlich die anderen genannten Komponenten enthält.

Durch die Verwendung vergleichsweise tiefer Temperaturen kann grundsätzlich der Gehalt an Ethan und Ethylen der gasförmigen ersten Fraktion deutlich reduziert werden, weil mehr Ethan und Ethylen in das oder die Kondensate übergehen.

Daher ist es bei der Verwendung vergleichsweise tiefer Temperaturen in einer ersten Ausführungsform auch möglich, nur das oder die Kondensate in der Tieftemperaturrektifikation zu bearbeiten, weil der nicht kondensierte Anteil des Trenneinsatzes nur geringe Mengen an Ethan und Ethylen enthält. In einer Druckwechseladsorption kann in diesem Fall lediglich die mittels der Tieftemperaturrektifikation aus den Kondensaten gebildete erste Fraktion bearbeitet werden. Die Menge an dem in der Druckwechseladsorption zu bearbeitenden Gas ist daher im Vergleich zu anderen Ausführungsformen vergleichsweise gering. Details sind insbesondere unter Bezugnahme auf die Figur 2 erläutert.

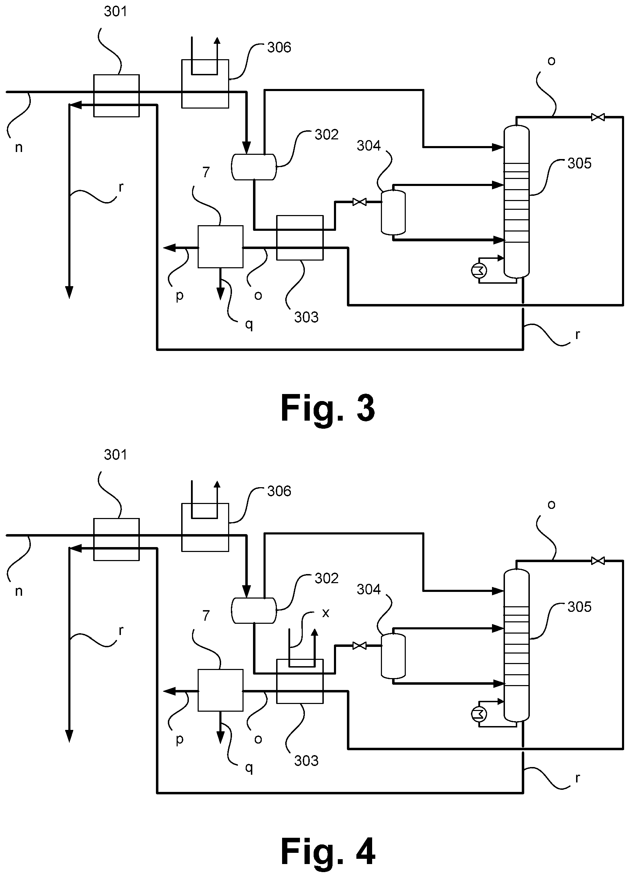

Wird hingegen, in einer zweiten Ausführungsform, in der Tieftemperaturtrennung eine Kühlung im ähnlichen Druckbereich wie in der ersten Ausführungsform, aber auf ein deutlich höheres Temperaturniveau vorgenommen, kondensieren weniger Ethan und Ethylen aus, so dass auch hier vorzugsweise die nicht kondensierten Anteile des Trenneinsatzes der Tieftemperaturrektifikation unterworfen werden, wie insbesondere unter Bezugnahme auf die Figur 3 erläutert. Typischerweise ist hierbei ferner der Gehalt an Ethan und Ethylen in der ersten Fraktion höher und wird in der sich anschließenden Druckwechseladsorption entsprechend weitgehend reduziert. In der Druckwechseladsorption muss daher ggf. eine im Vergleich zur Verwendung tieferer Temperaturen eine größere Gasmenge bearbeitet werden.

In einer dritten Ausführungsform, wie sie insbesondere unter Bezugnahme auf Figur 4 erläutert wird, wird in der Tieftemperaturtrennung eine Kühlung des Trenneinsatzes vorteilhafterweise in einem oder mehrere Abkühlschritte im ähnlichen Temperaturbereich wie in der ersten Ausführungsform, aber bei niedrigerem Druck vorgenommen. Ein nach den Abkühlschritten als Kondensat abgeschiedener Anteil des Trenneinsatzes wird zumindest teilweise entspannt und der Tieftemperaturrektifikation zugeführt. Die durch die Kühlung abgeschiedene Flüssigfraktion dient als Rücklauf in der Tieftemperaturrektifikation.

In jedem Fall, also den drei erläuterten Ausführungsformen, kann das Prozessgas bzw. der Trenneinsatz stromauf der Tieftemperaturtrennung einer Verdichtung unterworfen werden, wobei die überwiegend oder ausschließlich Ethan und Ethylen enthaltende dritte Fraktion zumindest zum Teil mit dem Prozessgas bzw. dem Trenneinsatz vereinigt und zusammen mit dem Prozessgas bzw. dem Trenneinsatz der Verdichtung unterworfen werden kann. Details zu den bei der Verdichtung erzielten Druckniveaus in unterschiedlichen Ausführungsformen sind unten erläutert. Auf diese Weise kann Ethan und Ethylen der Tieftemperaturtrennung erneut zugeführt und dabei insbesondere in der flüssigen zweiten Fraktion stromab der erläuterten Tieftemperaturrektifikation einer weiteren Tieftemperaturrektifikation zur Trennung von Ethan und Ethylen, also einem sogenannten Splitter, zugeführt werden. Das dabei gewonnene Ethylen kann als Produkt ausgeführt, das Ethan hingegen zu dem oder den verwendeten Reaktoren zurückgeführt werden.

Ebenfalls kann in den drei Ausführungsformen grundsätzlich eine Wärmeintegration dahingehend vorgenommen werden, dass der Trenneinsatz in der Tieftemperaturtrennung zumindest teilweise durch Übertragen von Wärme auf die gasförmige erste Fraktion und/oder die flüssige zweite Fraktion abgekühlt wird. Auf diese Weise kann gleichzeitig beispielsweise die gasförmige erste Fraktion auf eine für die Druckwechseladsorption geeignete Temperatur gebracht werden. Auch hierzu sind Details jeweils unten erläutert.

In der ersten Ausführungsform der vorliegenden Erfindung wird, wie erwähnt, der Trenneinsatz in der Tieftemperaturtrennung zum Abscheiden des oder der Kondensate auf vergleichsweise niedrige Temperaturniveaus, beispielsweise auf ein Temperaturniveau von -20 bis -100 °C, insbesondere -60 bis -100 °C, beispielsweise -80 bis -100 °C, abgekühlt. Die Kondensate werden, mit anderen Worten, auf einem entsprechenden Temperaturniveau abgeschieden. Entsprechende Temperaturniveaus können beispielsweise unter Verwendung von C2-Kältemittel, also insbesondere Ethylen, in einem Kältekreislauf bekannter Art erreicht werden. Entsprechende tiefe Temperaturen können auch dadurch erzielt werden, dass die in der Tieftemperaturrektifikation gebildete gasförmige erste Fraktion entspannt wird. Auf diese Weise können auch nochmals tiefere Temperaturen erzielt werden, allerdings nur unter Einsatz entsprechend aufwendiger Maschinen. Im Rahmen der Erfindung kann durch die Verwendung der Druckwechseladsorption grundsätzlich auf diese aufwendigen Maßnahmen verzichtet werden.

In einer Ausführungsform der vorliegenden Erfindung, insbesondere der zuvor erläuterten ersten Ausführungsform, kann die Tieftemperaturrektifikation bei Verwendung derartig niedriger Temperaturen unter Verwendung eines flüssigen Rücklaufs durchgeführt werden, der durch Kondensieren eines Teils der gasförmigen ersten Fraktion, die in diesem Fall Methan enthält, gebildet wird. Auf diese Weise kann der Gehalt an Ethan und Ethylen in der gasförmigen ersten Fraktion verringert werden.

Durch die Druckwechseladsorption kann Ethylen zurückgewonnen und Expander, die ansonsten ggf. für die Produktion von Spitzenkälte aus der gasförmigen ersten Fraktion installiert werden würden, um auch letzte Reste an Ethan und Ethylen zurückgewinnen zu können, können entfallen. Die gasförmige erste Fraktion kann auch in diesem Fall für die Wärmeintegration genutzt werden und der ethylenfreie Restgasstrom, d.h. die eine überwiegend oder ausschließlich tiefer als Ethan und Ethylen siedende Verbindungen enthaltende vierte Fraktion aus der Druckwechseladsorption, kann beispielsweise thermisch verwertet oder anderweitig genutzt werden. Durch die geringeren Mengen an Ethan und Ethylen in der gasförmigen ersten Fraktion kann die Druckwechseladsorption in dieser Ausführungsform, wie bereits erwähnt, grundsätzlich wesentlich kleiner als in einer Ausführungsform, in der höhere Temperaturniveaus verwendet werden, ausgeführt werden.

Werden in der Tieftemperaturtrennung zum Abscheiden des oder der Kondensate höhere Temperaturniveaus eingesetzt, betragen diese vorteilhafterweise nicht weniger als -20 bis -40 °C, insbesondere -30 bis -40 °C, beispielsweise -35 bis -40 °C. Die Kondensate werden also auf Temperaturniveaus abgeschieden, die nicht geringer als die genannten Werte sind. Dies schließt nicht aus, dass die Kondensate selbst anschließend nicht noch weiteren Abkühlschritten zugeführt werden können, wie dies beispielsweise in der in Figur 3 veranschaulichten zweiten Ausführungsform der Fall ist. Diese Temperaturniveaus lassen sich durch die Verwendung von C3-Kältemittel, also insbesondere Propylen, erzielen. Aufgrund der deutlich höheren Temperaturen als zuvor erläutert verringern sich dabei der apparative Aufwand und der Materialaufwand. Allerdings sind die dann ggf. in größeren Mengen in die gasförmige erste Fraktion übergehenden Mengen an Ethan und Ethylen ggf. in einer entsprechend größer dimensionierten Druckwechseladsorption abzutrennen. Die jeweiligen Vorteile der niedrigeren oder höheren Temperaturniveaus sind je nach den gegebenen Umständen, beispielsweise dem Vorhandensein eines (C2- oder C3-)Kältekreislaufs, abzuwägen. Bei der Verwendung von höheren Temperaturniveaus zur Abscheidung der Kondensate wie zuvor erläutert kann der Trenneinsatz vor dem Abkühlen in der zweiten Ausführungsform der vorliegenden Erfindung auf ein Druckniveau von 25 bis 35 bar, beispielsweise ca. 30 bar, verdichtet werden. Die Tieftemperaturrektifikation kann dann auf einem Druckniveau von auf ein Druckniveau von 15 bis 25 bar, beispielsweise ca. 19 bar, durchgeführt, auf dem auch die Tieftemperaturrektifikation betrieben wird. Das oder die Kondensate, die der Tieftemperaturrektifikation unterworfen werden, oder deren der Tieftemperaturrektifikation unterworfener Teil wird bzw. werden dann auf das Druckniveau der Tieftemperaturrektifikation entspannt, bevor sie der Tieftemperaturrektifikation zugeführt wird oder werden.

Bei der Verwendung von höheren Temperaturniveaus zur Abscheidung der Kondensate wie zuvor erläutert kann der Trenneinsatz aber alternativ zu der soeben erläuterten zweiten Ausführungsform in der dritten Ausführungsform der vorliegenden Erfindung vor dem Abkühlen auf ein Druckniveau von 20 bis 25 bar, insbesondere ca. 22 bar, verdichtet werden. Die Tieftemperaturrektifikation kann dann auf einem Druckniveau von 9 bis 16 bar, insbesondere von ca. 13 bar, betrieben werden. Das oder die Kondensate, die der Tieftemperaturrektifikation unterworfen werden, oder deren der Tieftemperaturrektifikation unterworfener Teil wird bzw. werden dabei auch hier auf das Druckniveau der Tieftemperaturrektifikation entspannt, bevor sie der Tieftemperaturrektifikation zugeführt wird oder werden. Auf diese Weise kann ein Zweiphasengemisch erhalten werden, das in eine Gasphase und eine Flüssigphase getrennt wird. Die Gasphase und die Flüssigphase können jeweils zumindest zum Teil der Tieftemperaturrektifikation zugeführt und dabei beispielsweise in unterschiedlichen Höhen in eine Rektifikationskolonne eingespeist werden. Durch die Entspannung kann zusätzliche Kälte generiert werden.

Bei Einsatz der erläuterten vergleichsweise höheren Drücke ist eine zufriedenstellende Tieftemperaturrektifikation bei vergleichsweise höheren Temperaturen möglich, so dass das oder die Kondensate in geringerem Umfang abgekühlt werden müssen. Umgekehrt ist bei Einsatz der erläuterten vergleichsweise geringeren Drücke zwar eine tiefere Temperatur erforderlich, allerdings können die beteiligten Anlagenteile kostengünstiger, da weniger druckfest, ausgeführt werden können. Daher kann in bestimmten Szenarien die eine oder die andere Ausführungsform vorteilhaft sein.

Insbesondere kann in der zweiten und dritten Ausführungsform, bei denen die vergleichsweise höheren Temperaturniveaus eingesetzt werden, wie erwähnt auch der bei der Kondensation gasförmig verbleibende Rest des Trenneinsatzes oder ein Teil hiervon der Tieftemperaturrektifikation zugeführt werden

Unabhängig von den eingesetzten Druckniveaus kann der Trenneinsatz in der zweiten und dritten Ausführungsform der vorliegenden Erfindung durch Übertragen von Wärme auf die flüssige zweite Fraktion abgekühlt werden. Das Abkühlen des Trenneinsatzes umfasst also insbesondere das Übertragen von Wärme auf die zweite Fraktion.

Die Abkühlung des Trenneinsatzes auf das erwähnte höhere Temperaturniveau zum Abscheiden der Kondensate kann ferner die Verwendung geeigneter Kältemittel umfassen. Insbesondere eignet sich ein C3-Kältemittel wie Propylen, wodurch sich das Temperaturniveau von -20 bis -40 °C erreichen lässt.

Wie erwähnt können das oder die Kondensate in den hier erläuterten Ausführungsformen, insbesondere vor der Entspannung, weiter abgekühlt werden. Je nach den zuvor beschriebenen Druckniveaus können dabei unterschiedliche Temperaturniveaus zum Einsatz kommen. So kann bzw. können bei dem erläuterten höheren Druckniveaus des Trenneinsatzes und der Rektifikation gemäß der zweiten Ausführungsform das oder die Kondensate oder deren der Tieftemperaturrektifikation unterworfener Teil durch Übertragen von Wärme auf die erste Fraktion weiter auf ein Temperaturniveau von -30 bis -50 °C abgekühlt werden. Bei den erläuterten tieferen Druckniveaus gemäß der dritten Ausführungsform kann bzw. können hingegen das oder die Kondensate oder deren der Tieftemperaturrektifikation unterworfener Teil durch Übertragen von Wärme auf die erste Fraktion und ein Kältemittel auf ein Temperaturniveau von -90 bis -100 °C weiter abgekühlt werden.

Vorteilhafterweise wird die gasförmige erste Fraktion zur Abkühlung des oder der Kondensate oder deren jeweiliger Teile eingesetzt, in dem jedoch insbesondere dann, wenn die genannten tiefen Temperaturen erzielt werden sollen, auch ein weiteres Kältemittel, insbesondere ein C2-Kältemittel wie Ethylen, eingesetzt wird. Die gasförmige erste Fraktion kann vor der Verwendung zur Abkühlung des oder der Kondensate oder deren Teilen auch insbesondere entspannt und nach der Verwendung zur Abkühlung der Druckwechseladsorption zugeführt werden.

Beschrieben wird auch eine Anlage zur Herstellung von Ethylen, die dafür eingerichtet ist, unter Einsatz einer Dehydrierung von Ethan ein Prozessgas zu bilden, das zumindest Ethan, Ethylen und tiefer als Ethan und Ethylen siedende Verbindungen enthält, und unter Verwendung zumindest eines Teils des Prozessgases einen Trenneinsatz zu bilden und einer Tieftemperaturtrennung zu unterwerfen, in welcher Mittel vorgesehen sind, die dafür eingerichtet sind, den Trenneinsatz

abzukühlen, ein oder mehrere Kondensate aus dem Trenneinsatz abzuscheiden, und zumindest das oder die Kondensate zumindest zum Teil unter Erhalt einer gasförmigen ersten Fraktion und einer flüssigen zweiten Fraktion einer Tieftemperaturrektifikation zu unterwerfen, wobei die Tieftemperaturrektifikation dafür eingerichtet ist, die gasförmige erste Fraktion derart zu bilden, dass diese zumindest das Ethan und das Ethylen in einem geringeren Anteil als in dem Trenneinsatz und die tiefer als Ethan und Ethylen siedenden Verbindungen einem höheren Anteil als in dem Trenneinsatz enthält.

Erfindungsgemäß sind Mittel vorgesehen, die dafür eingerichtet sind, die gasförmige erste Fraktion zumindest zum Teil einer Druckwechseladsorption zu unterwerfen, mittels welcher eine überwiegend oder ausschließlich Ethan und Ethylen enthaltende dritte Fraktion und eine überwiegend oder ausschließlich die tiefer als Ethan und Ethylen siedende Verbindungen enthaltende vierte Fraktion gebildet werden.

Zu Merkmalen und Vorteilen einer entsprechenden Anlage sei auf die obigen Erläuterungen zu den Merkmalen und Vorteilen des Verfahrens verwiesen. Insbesondere ist eine derartige Anlage zur Durchführung eines Verfahrens gemäß den oben erläuterten spezifischen Ausgestaltungen eingerichtet und weist hierzu geeignete Mittel auf. Auch diesbezüglich sei auf die obigen Ausführungen verwiesen.

### Kurze Beschreibung der Zeichnungen

Die Erfindung wird nachfolgend unter Bezugnahme auf die beigefügten Zeichnungen näher erläutert, die unter anderem bevorzugte Ausführungsformen der vorliegenden Erfindung veranschaulichen.
Figur 1 veranschaulicht eine Anlage zur Herstellung von Olefinen gemäß einer Ausführungsform der Erfindung.
Figur 2 veranschaulicht eine Tieftemperaturtrennung zum Einsatz in einer Anlage gemäß einer Ausführungsform der Erfindung.
Figur 3 veranschaulicht eine Tieftemperaturtrennung zum Einsatz in einer Anlage gemäß einer Ausführungsform der Erfindung.
Figur 4 veranschaulicht eine Tieftemperaturtrennung zum Einsatz in einer Anlage gemäß einer Ausführungsform der Erfindung.

### Ausführliche Beschreibung der Zeichnungen

In den nachfolgenden Figuren sind einander funktionell oder baulich entsprechende Elemente mit identischen Bezugszeichen angegeben und werden der Übersichtlichkeit halber nicht wiederholt erläutert. Werden nachfolgend Anlagen und Anlagenteile beschrieben, gelten die Erläuterungen zu diesen sinngemäß auch für die mittels dieser Anlagenteile implementierten Verfahrensschritte und umgekehrt.

In Figur 1 ist eine Anlage zur Herstellung von Olefinen gemäß einer Ausführungsform der Erfindung in Form eines stark vereinfachten Anlagendiagramms veranschaulicht und insgesamt mit 100 bezeichnet. Wenngleich nachfolgend eine Anlage 100 zur ODH von Ethan (ODH-E) beschrieben wird, eignet sich, wie erwähnt, die vorliegende Erfindung auch zum Einsatz bei einer nichtoxidativen Dehydrierung von Ethan. In diesem Fall gelten die nachfolgenden Erläuterungen entsprechend.

Der Anlage 100 wird ein ethanreicher Frischeinsatz a, der ggf. gewisse Mengen Methan enthält, mit einem ethanreichen, rückgeführten Stoffstrom b gemischt und einem oder mehreren ODH-E-Reaktoren 1 zugeführt. Dem oder den Reaktoren 1 wird dabei ferner ein Verdünnungsmedium c (beispielsweise Wasserdampf, Stickstoff oder Kohlendioxid) und Sauerstoff d zugeführt.

Die Hauptreaktion der ODH-E lautet:

C₂H₆ + ½ O₂ → C₂H₄ + H₂O (1)

Als Nebenreaktion treten vor allem die Bildung von Kohlenmonoxid, Kohlendioxid und auch von Essigsäure auf.

Ein in Form eines Stoffstroms e dem oder den Reaktoren 1 entnommenes Gasgemisch, hier als "Prozessgas" bezeichnet, wird einer Abkühlung 2 unterworfen und Wasser wird in Form eines Stoffstroms f abgetrennt. Essigsäure wird, falls vorhanden, ebenfalls aus dem Prozessgas abgetrennt und mit dem Stoffstrom f ausgeschleust. Der Stoffstrom f kann anschließend weiterbearbeitet werden, um Essigsäure zu gewinnen (nicht veranschaulicht).

Das abgekühlte Prozessgas wird in Form eines Stoffstroms g einer Verdichtung 3 unterworfen und verdichtet, i.d.R. in einem mehrstufigen Verdichter. Dabei wird zwischen den Verdichterstufen das Prozessgas in Form eines Stoffstroms h abgezogen, um Kohlendioxid in Form eines Stoffstroms k abzutrennen. Dies kann beispielsweise durch eine Aminwäsche, Pottaschwäsche oder Laugenwäsche in einer Kohlendioxidentfernungseinheit 4 geschehen. Auch können andere Verfahren wie Membranverfahren oder eine Kombination aus verschiedenen Verfahren eingesetzt werden. Das von Kohlendioxid befreite Prozessgas wird in Form eines Stoffstroms I wieder zurück zur Verdichtung 3 geleitet.

Das verdichtete Prozessgas wird in Form eines Stoffstroms m einer Trocknung 5, das entsprechend getrocknete Prozessgas in Form eines Stoffstroms n einer Tieftemperaturtrennung 6 zugeführt. Details einer Tieftemperaturtrennung 6 sind in Form von Ausführungsbeispielen in Figur 2, 3 und 4 veranschaulicht.

In der Tieftemperaturtrennung 6 wird das Prozessgas bzw. ein aus diesem gebildeter Trenneinsatz über ein oder mehrere Temperaturniveaus abgekühlt und es werden ein oder mehrere Kondensate aus dem Prozessgas abgeschieden, wobei das oder die Kondensate zumindest zum Teil unter Erhalt einer gasförmigen ("ersten") Fraktion und einer flüssigen ("zweiten") Fraktion einer Tieftemperaturrektifikation unterworfen werden, wobei die gasförmige erste Fraktion zumindest das Ethan und das Ethylen in einem geringeren Anteil als in dem Trenneinsatz und zumindest die tiefer als Ethan und Ethylen siedenden Verbindungen in einem höheren Anteil als in dem Trenneinsatz enthält. Das Prozessgas wird also stufenweise abgekühlt und anfallendes Kondensat wird einer Rektifikationskolonne zugeführt.

Am Kopf der Rektifikationskolonne wird die erwähnte gasförmige erste Fraktion in Form eines Stoffstroms o abgezogen. Zur Bildung des Stoffstroms o kann auch ein bei der erwähnten stufenweisen Abkühlung gasförmig verbleibender Anteil des Prozessgases verwendet werden. In diesem Stoffstrom o sind aufgrund der erwähnten geringen Mengen des als Rücklauf in der Tieftemperaturrektifikation verwendbaren Methans auch noch beträchtliche Mengen Ethan und Ethylen vorhanden.

Um die Verluste an Produkt (Ethylen) und Edukt (Ethan) zu reduzieren, wird in der in Figur 1 dargestellten Anlage 100 eine Druckwechseladsorption 7 zur Rückgewinnung von Ethylen (und auch Ethan) vorgeschlagen. Dabei werden aus dem Stoffstrom o Ethan und Ethylen an einem geeigneten Adsorbens adsorbiert. Hierbei adsorbieren bevorzugt Ethan und Ethylen im Gegensatz zu den leichten Gasen Stickstoff und Argon, die insbesondere als Verunreinigungen im Sauerstoff d in die Anlage 100 eingebracht werden, aber auch im Gegensatz zu Sauerstoff, Kohlenmonoxid, Methan und anderen leichten Gase wie Wasserstoff.

Ethan und Ethylen werden bei geringerem Druck in Form einer überwiegend Ethan und Ethylen enthaltenden ("dritten") Fraktion wieder abgegeben und können in Form eines Stoffstroms p abgezogen werden. Der Stoffstrom p kann insbesondere zur Verdichtung 3 rezykliert werden. Der von Ethylen und Ethan befreite Stoffstrom o, nun mit q bezeichnet, also eine überwiegend oder ausschließlich tiefer als Ethan und Ethylen siedende Komponenten enthaltende ("vierte") Fraktion steht noch unter hohem Druck und kann zur Gewinnung mechanischer Arbeit und thermischer Energie oder als Exportstrom für andere Anwendungen genutzt werden. Vor der Adsorption kann der Stoffstrom o auch zur Kälterückgewinnung genutzt werden.

Die Grundidee der vorliegenden Erfindung besteht in einer Kombination einer Tieftemperaturtrennung 6 mit einer Druckwechseladsorption 7. Hierbei können unterschiedliche Ausführungsformen vorgesehen sein. In einer Ausführungsform, die in Figur 2 veranschaulicht ist, wird eine Kühlung des Prozessgases auf ein ausgesprochen tiefes Temperaturniveau vorgenommen, so dass der Gehalt an Ethan und Ethylen in dem Stoffstrom o, der anschließend der Druckwechseladsorption unterworfen wird, reduziert wird. In weiteren Ausführungsformen, die in Figur 3 und 4 veranschaulicht sind, wird eine Kühlung auf ein deutlich höheres Temperaturniveau vorgenommen, so dass der Gehalt an Ethan und Ethylen erst in der Druckwechseladsorption entsprechend reduziert wird.

In der Tieftemperaturtrennung 6 verbleibt die flüssige zweite Fraktion, die in Form eines Stoffstroms r aus der Tieftemperaturtrennung 6 abgezogen werden kann. Diese flüssige zweite Fraktion enthält überwiegend oder ausschließlich Ethylen und Ethan. Der Stoffstrom r wird im dargestellten Beispiel in einem Splitter 8 zugeführt, der ebenfalls eine Tieftemperaturrektifikation umfasst. In dem Splitter 8 wird ein überwiegend oder ausschließlich Ethylen enthaltendes Ethylenprodukt gewonnen und in Form eines Stoffstroms s abgezogen. Eine ebenfalls in dem Splitter 8 gebildete, überwiegend oder ausschließlich Ethan enthaltende Fraktion kann in Form des Stoffstroms b zu dem oder den Reaktoren rezykliert werden.

Falls eine schwere Fraktion an Kohlenwasserstoffen mit drei und mehr Kohlenstoffatomen existiert, kann diese stromauf oder stromab der Tieftemperaturtrennung 6 abgetrennt werden. Je nach Menge können diese schweren Kohlenwasserstoffe auch aus dem Stoffstrom b abgetrennt werden.

Figur 2 veranschaulicht eine Tieftemperaturtrennung zum Einsatz in einer Anlage zur Herstellung von Olefinen, wie sie beispielsweise in der in Figur 1 gezeigten Anlage 100 als Tieftemperaturtrennung 6 eingesetzt werden kann. Diese Tieftemperaturtrennung, die der zuvor erläuterten ersten Ausführungsform entspricht, umfasst eine stufenweise Abkühlung. Die bereits in Figur 1 dargestellten Stoffströme n, o und r sind zur Veranschaulichung der Einbindung der in Figur 2 gezeigten Tieftemperaturtrennung in eine entsprechende Anlage 100 auch hier gezeigt. Die Darstellung der jeweiligen Elemente ist nicht positions- und nicht maßstabsgerecht.

Das Prozessgas wird in Form des Stoffstroms n der Tieftemperaturtrennung zugeführt. Das Prozessgas wird nacheinander durch Wärmetauscher 201 bis 204 geführt und dort auf immer niedrigere Temperaturniveaus gekühlt. Die Wärmetauscher 201 bis 204 können dazu mit nicht veranschaulichten Ethylenströmen gekühlt werden. Zur Kühlung kann, wie ebenfalls nicht gesondert veranschaulicht, zusätzlich der Stoffstrom o eingesetzt werden, also die mehrfach erläuterte gasförmige erste Fraktion.

Stromab der Wärmetauscher 201 bis 204 wird das Prozessgas bzw. ein durch die Abkühlung in den Wärmetauschern 201 bis 204 jeweils gebildetes Zweiphasengemisch jeweils in Abscheider 205 bis 208 überführt, wo sich jeweils ein Kondensat aus dem Prozessgas abscheidet. Die Kondensate werden in einer ihrer stofflichen Zusammensetzung entsprechender Höhe in eine Rektifikationskolonne 209 eingespeist. Ein geringerer Anteil des Prozessgases des Stoffstroms n kann auch, wie hier nicht veranschaulicht, direkt in die Rektifikationskolonne 209 eingespeist werden.

Ein Sumpfverdampfer 210 der Rektifikationskolonne 209 wird beispielsweise unter Verwendung von Propan beheizt, ein Kopfkondensator 211 beispielsweise unter Verwendung von Niederdruckethylen gekühlt. Die Rektifikationskolonne 209 wird derart betrieben, dass sich an ihrem Kopf überwiegend tiefer als Ethan und Ethylen siedende Komponenten und in ihrem Sumpf die höher siedenden Verbindungen anreichern. Auf diese Weise kann vom Kopf der Rektifikationskolonne 209 ein Teil des Stoffstroms o, hier mit o1 bezeichnet, und aus dem Sumpf der Rektifikationskolonne 7 der Stoffstrom r abgezogen werden. Ein in dem Abscheider 208 gasförmig verbliebener Anteil des Prozessgases, hier in Form eines Stoffstroms o2 veranschaulicht, kann ebenfalls bei der Bildung des Stoffstroms o verwendet werden.

Die Temperatur des Prozessgases bzw. stromab des Wärmetauschers 201 beträgt beispielsweise ca. -30 °C, die Temperatur stromab des Wärmetauschers 202 beispielsweise ca. -50 °C, die Temperatur stromab des Wärmetauschers 203 beispielsweise ca. -75 °C und die Temperatur stromab des Wärmetauschers 204 beispielsweise ca. -99 °C. Der Sumpfverdampfer 210 wird auf einem Temperaturniveau von beispielsweise ca. -17 °C, der Kopfkondensator 211 auf einem Temperaturniveau von beispielsweise ca. -97 °C betrieben.

Durch den Einsatz einer entsprechenden Kältemaschine, beispielsweise mit dem Kältemittel Ethylen, sind entsprechend tiefe Temperaturen erreichbar. Auf diese Weise kann der Anteil an Ethylen in der gasförmigen ersten Fraktion, also dem Stoffstrom o, wesentlich reduziert werden. Der Einsatz einer Druckwechseladsorption 7 zur Abtrennung von Ethylen kann je nach Zusammensetzung des Prozessgases bzw. des Stoffstroms n dennoch energetisch und wirtschaftlich sinnvoll sein.

In diesem Fall werden durch die Druckwechseladsorption Ethan und Ethylen zurückgewonnen und Expander, die ansonsten ggf. für die Produktion von Spitzenkälte aus dem Stoffstrom o installiert werden würden, können entfallen. Der Stoffstrom o kann weiterhin für die Wärmeintegration genutzt werden und der ethylenfreie Restgasstrom q (siehe Figur 1) kann wie im vorherigen Fall verwertet werden. Zudem kann die Druckwechseladsorption wesentlich kleiner ausgeführt werden, als wenn keine Abkühlung auf entsprechend tiefe Temperaturniveaus vorgenommen wird.

Figur 3 veranschaulicht eine Tieftemperaturtrennung zum Einsatz in einer Anlage zur Herstellung von Olefinen, wie sie ebenfalls beispielsweise in der in Figur 1 gezeigten Anlage 100 als Tieftemperaturtrennung 6 eingesetzt werden kann. Diese Tieftemperaturtrennung, die mit den zuvor erläuterten höheren Temperaturniveaus arbeitet, umfasst eine mehrstufige Abkühlung auf ein deutlich höheres Temperaturniveau als zu Figur 2 erläutert. Auch hier sind die in Figur 1 dargestellten Stoffströme n, o und r zur Veranschaulichung der Einbindung der in Figur 3 gezeigten Tieftemperaturtrennung in eine entsprechende Anlage 100 gezeigt. Zusätzlich sind hier auch die Druckwechseladsorption 7 und die Stoffströme p und q gezeigt.

Das Prozessgas wird auf einem Temperaturniveau von beispielsweise ca. -9 °C und auf einem Druckniveau von beispielsweise ca. 30 bar in die Tieftemperaturtrennung eingespeist und durch einen ersten Wärmetauscher 301 geführt, der unter Verwendung des Stoffstroms r, also der flüssigen zweiten Fraktion, gekühlt werden kann. Nach der Abkühlung in dem ersten Wärmetauscher 301 wird das Prozessgas dann in einem zweiten Wärmetauscher 306 auf ein Temperaturniveau von beispielsweise ca. -35 °C abgekühlt. Auf diese Weise wird ein Zweiphasenstrom gebildet, der in einen Abscheidebehälter 302 eingespeist wird. In dem Abscheidebehälter 302 bilden sich eine flüssige und eine gasförmige Fraktion. Die gasförmige Fraktion wird entspannt und in eine Rektifikationskolonne 305 eingespeist. Die Entspannung in den zweiten Abscheidebehälter 304 erfolgt dabei von dem Druckniveau von beispielsweise ca. 30 bar auf ein Druckniveau von beispielsweise ca. 19 bar, auf dem auch die Rektifikationskolonne 305 betrieben wird. Der Wärmetauscher 306 kann dabei beispielsweise mit Niederdruckpropylen oder einem entsprechenden C3-Kältemittel betrieben werden.

Die flüssige Fraktion wird dem ersten Abscheidebehälter 302 entnommen und in einem dritten Wärmetauscher 303 auf ein Temperaturniveau von beispielsweise ca. -38 °C abgekühlt und auf dem Druckniveau, bei dem die Rektifikationskolonne betrieben wird in einen zweiten Abscheidebehälter 304 entspannt. Dabei bilden sich eine flüssige und eine gasförmige Fraktion, die in die Reaktifikationskolonne eingespeist werden. Die flüssige Fraktion wird als Rücklauf auf die Rektifikationskolonne 305 verwendet. Auch die gasförmige Fraktion aus dem ersten Abscheidebehälter 302 wird in die Rektifikationskolonne eingespeist.

Die Rektifikationskolonne 305 wird unter Verwendung eines Sumpfverdampfers betrieben, der beispielsweise mit Niederdruckpropylen betrieben werden kann. Der Stoffstrom r kann der Rektifikationskolonne 305 auf einem Temperaturniveau von beispielsweise ca. -21 °C entnommen werden. Vom Kopf der Rektifikationskolonne 305 wird der Stoffstrom o auf einem Temperaturniveau von beispielsweise ca. -45 °C abgezogen, weiter entspannt und durch den zweiten Wärmetauscher 303 geführt. Der Stoffstrom o, also die gasförmige zweite Fraktion, wird anschließend in die Druckwechseladsorption 7 eingespeist. Hierzu und der weiteren Behandlung der Stoffströme p und q wird auf die Erläuterungen zu Figur 1 verwiesen.

Figur 4 veranschaulicht eine Tieftemperaturtrennung zum Einsatz in einer Anlage zur Herstellung von Olefinen, wie sie ebenfalls beispielsweise in der in Figur 1 gezeigten Anlage 100 als Tieftemperaturtrennung 6 eingesetzt werden kann. Diese Tieftemperaturtrennung stellt eine Variante der in Figur 3 veranschaulichten Tieftemperaturtrennung dar, die sich im Wesentlichen durch die verwendeten Drücke und Temperaturen unterscheidet. Daher sind die gezeigten Elemente mit identischen Bezugszeichen versehen.

Auch hier erfolgt eine Abkühlung in den Wärmetauschern 301 und 306 auf die erläuterten vergleichsweise hohen Temperaturniveaus. Jedoch wird der Trenneinsatz der Tieftemperaturtrennung auf beispielsweise ca. 22 bar zugeführt und die Tieftemperaturrektifikation auf beispielsweise ca. 13 bar durchgeführt. Die flüssige Fraktion, die dem ersten Abscheidebehälter 302 entnommen wird, wird hier in dem dritten Wärmetauscher 303 durch den zusätzlichen Einsatz eines geeigneten Kältemittels un Form eines Stoffstroms auf ein Temperaturniveau von beispielsweise ca. 97 °C abgekühlt. Auf diese Weise kann der Stoffstrom r der Rektifikationskolonne 305 auf einem Temperaturniveau von beispielsweise ca. -35 °C entnommen werden. Vom Kopf der Rektifikationskolonne 305 wird der Stoffstrom o auf einem Temperaturniveau von beispielsweise ca. -97 °C abgezogen, weiter entspannt und durch den zweiten Wärmetauscher 303 geführt.

## Patentansprüche

1. Verfahren zur Herstellung von Ethylen, bei dem unter Einsatz einer Dehydrierung von Ethan ein Prozessgas gebildet wird, das zumindest Ethan, Ethylen und tiefer als Ethan und Ethylen siedende Verbindungen enthält, bei dem unter Verwendung zumindest eines Teils des Prozessgases ein Trenneinsatz gebildet und einer Tieftemperaturtrennung (6) unterworfen wird, in welcher der Trenneinsatz abgekühlt wird und in welcher ein oder mehrere Kondensate aus dem Trenneinsatz abgeschieden werden, und bei dem zumindest ein Teil des oder der Kondensate unter Erhalt einer gasförmigen ersten Fraktion und einer flüssigen zweiten Fraktion einer Tieftemperaturrektifikation unterworfen werden, wobei die gasförmige erste Fraktion zumindest das Ethan und das Ethylen in einem geringeren Anteil als in dem Trenneinsatz und die tiefer als Ethan und Ethylen siedenden Verbindungen in einem höheren Anteil als in dem Trenneinsatz enthält, **dadurch gekennzeichnet, dass** die erste Fraktion zumindest zum Teil einer Druckwechseladsorption (7) unterworfen wird, mittels welcher eine überwiegend oder ausschließlich Ethylen und Ethan enthaltende dritte Fraktion und eine überwiegend oder ausschließlich Methan und Kohlenmonoxid enthaltende vierte Fraktion gebildet werden.

2. Verfahren nach einem der vorstehenden Ansprüche, bei dem der Trenneinsatz in der Tieftemperaturtrennung (6) zum Abscheiden des oder der Kondensate auf ein Temperaturniveau von -40 bis -100 °C abgekühlt wird .

3. Verfahren nach Anspruch 2, bei dem die Tieftemperaturrektifikation unter Verwendung eines flüssigen Rücklaufs durchgeführt wird, der durch Kondensieren eines Teils der ersten Fraktion gebildet wird.

4. Verfahren nach Anspruch 1, bei dem bei dem der Trenneinsatz in der Tieftemperaturtrennung (6) zum Abscheiden des oder der Kondensate auf ein Temperaturniveau von -20 bis -40 °C abgekühlt wird.

5. Verfahren nach Anspruch 4, bei dem der Trenneinsatz vor dem Abkühlen auf ein Druckniveau von 25 bis 35 bar verdichtet wird und die Tieftemperaturrektifikation auf einem Druckniveau von 15 bis 25 bar betrieben wird.

6. Verfahren nach Anspruch 4, bei dem der Trenneinsatz vor dem Abkühlen auf ein Druckniveau von 20 bis 25 bar verdichtet wird und die Tieftemperaturrektifikation auf einem Druckniveau von 9 bis 16 bar betrieben wird.

7. Verfahren nach einem der Ansprüche 4 bis 6, bei dem das Abkühlen des Trenneinsatzes das Übertragen von Wärme auf die zweite Fraktion umfasst.

8. Verfahren nach Anspruch 7, bei dem das Abkühlen des Trenneinsatzes das Übertragen von Wärme auf ein Kältemittel umfasst.

9. Verfahren nach Anspruch 5, bei dem das oder die Kondensate oder deren der Tieftemperaturrektifikation unterworfener Teil durch Übertragen von Wärme auf die erste Fraktion weiter auf ein Temperaturniveau von -30 bis -30°C abgekühlt wird oder werden.

10. Verfahren nach Anspruch 6, bei dem das oder die Kondensate oder deren der Tieftemperaturrektifikation unterworfener Teil durch Übertragen von Wärme auf die erste Fraktion und ein Kältemittel auf ein Temperaturniveau von -40 bis -100 °C weiter abgekühlt wird oder werden.

11. Verfahren nach Anspruch 9 oder 10, bei dem die erste Fraktion entspannt wird, bevor Wärme des oder der Kondensate oder deren der Tieftemperaturrektifikation unterworfenen Teils auf sie übertragen wird.

12. Verfahren nach Anspruch 11, bei dem die erste Fraktion nach dem Übertragen der Wärme der Druckwechseladsorption (7) zugeführt wird.

13. Verfahren nach einem der Ansprüche 4 bis 12, bei dem auch ein bei der Bildung der Kondensate gasförmig verbleibender Anteil des Trenneinsatzes zumindest teilweise der Tieftemperaturrektifikation zugeführt wird.

## Claims

1. Method for the production of ethylene, in which a process gas is formed using a dehydrogenation of ethane, which process gas contains at least ethane, ethylene, and compounds having a lower boiling point than ethane and ethylene, in which a fractional charge is formed using at least a portion of the process gas and is subjected to a cryogenic separation (6), in which the fractional charge is cooled, and in which one or more condensates are precipitated from the fractional charge, and in which at least a portion of the one or more condensates is subjected to a cryogenic rectification, with yield of a gaseous first fraction and a liquid second fraction, wherein the gaseous first fraction contains at least the ethane and ethylene in a smaller proportion than in the fractional charge and contains the compounds having a lower boiling point than ethane and ethylene in a larger proportion than in the fractional charge, **characterized in that** the first fraction is subjected at least in part to a pressure swing adsorption (7) by means of which a third fraction predominantly or exclusively containing ethylene and ethane and a fourth fraction predominantly or exclusively containing methane and carbon monoxide are formed.

2. Method according to one of the preceding claims, in which the fractional charge is cooled to a temperature level of -40 to -100 °C in the cryogenic separation (6) in order to precipitate the condensate or condensates.

3. Method according to claim 2, in which the cryogenic rectification is performed using a liquid reflux that is formed by condensing a portion of the first fraction.

4. Method according to claim 1, in which the fractional charge is cooled to a temperature level of -20 to -40 °C in the cryogenic separation (6) in order to precipitate the condensate or condensates.

5. Method according to claim 4, in which the fractional charge is compressed to a pressure level of 25 to 35 bar before cooling, and the cryogenic rectification is operated at a pressure level of 15 to 25 bar.

6. Method according to claim 4, in which the fractional charge is compressed to a pressure level of 20 to 25 bar before cooling, and the cryogenic rectification is operated at a pressure level of 9 to 16 bar.

7. Method according to one of claims 4 through 6, in which the cooling of the fractional charge comprises transferring heat to the second fraction.

8. Method according to claim 7, in which the cooling of the fractional charge comprises transferring heat to a refrigerant.

9. Method according to claim 5, in which the condensate or condensates, or the portion thereof subjected to cryogenic rectification, is or will be further cooled to a temperature level of -30 to -30 °C by transferring heat to the first fraction.

10. Method according to claim 6, in which the condensate or condensates, or the portion thereof subjected to cryogenic rectification, is or will be further cooled to a temperature level of -40 to -100 °C by transferring heat to the first fraction and a refrigerant.

11. Method according to claim 9 or 10, in which the first fraction is relaxed prior to heat of the condensate or condensates, or of their portion subjected to cryogenic rectification, being transferred to it.

12. Method according to claim 11, in which the first fraction is supplied to the pressure swing adsorption (7) after the transfer of heat.

13. Method according to one of claims 4 through 12, in which a portion of the fractional charge which remains gaseous upon formation of the condensates is also supplied at least in part to the cryogenic rectification.

## Revendications

1. Procédé de fabrication d'éthylène, dans lequel un gaz de procédé est formé par déshydrogénation d'éthane, qui contient au moins de l'éthane, de l'éthylène et des composés à point d'ébullition inférieur à l'éthane et à l'éthylène, dans lequel un insert de séparation est formé en utilisant au moins une partie du gaz de procédé et est soumis à une séparation cryogénique (6), dans lequel l'insert de séparation est refroidi et dans lequel un ou plusieurs condensats sont isolés de l'insert de séparation, et dans lequel au moins une partie du ou des condensats est soumise à une rectification à basse température pour obtenir une première fraction gazeuse et une deuxième fraction liquide, la première fraction gazeuse contenant au moins l'éthane et l'éthylène dans une proportion inférieure à celle de l'insert de séparation et les composés dont le point d'ébullition est inférieur à l'éthane et l'éthylène dans une proportion plus élevée que dans l'insert de séparation, **caractérisé en ce que** la première fraction est soumise au moins en partie à une adsorption modulée en pression (7), au moyen de laquelle sont formées une troisième fraction contenant principalement ou exclusivement de l'éthylène et de l'éthane et une quatrième fraction contenant principalement ou exclusivement du méthane et du monoxyde de carbone.

2. Procédé selon l'une des revendications précédentes, dans lequel l'insert de séparation est refroidi dans la séparation cryogénique (6) à un niveau de température allant de -40 à -100 °C pour isoler le ou les condensats.

3. Procédé selon la revendication 2, dans lequel la rectification à basse température est effectuée en utilisant un reflux liquide formé en condensant une partie de la première fraction.

4. Procédé selon la revendication 1, dans lequel l'insert de séparation est refroidi dans la séparation cryogénique (6) à un niveau de température allant de - 20 à -40 °C pour isoler le ou les condensats.

5. Procédé selon la revendication 4, dans lequel l'insert de séparation est comprimé avant refroidissement à un niveau de pression allant de 25 à 35 bar et la rectification à basse température est effectuée à un niveau de pression allant de 15 à 25 bar.

6. Procédé selon la revendication 4, dans lequel l'insert de séparation est comprimé avant refroidissement à un niveau de pression allant de 20 à 25 bar et la rectification à basse température est effectuée à un niveau de pression allant de 9 à 16 bar.

7. Procédé selon l'une quelconque des revendications 4 à 6, dans lequel le refroidissement de l'insert de séparation comprend le transfert de chaleur à la deuxième fraction.

8. Procédé selon la revendication 7, dans lequel le refroidissement de l'insert de séparation comprend le transfert de chaleur à un fluide frigorigène.

9. Procédé selon la revendication 5, dans lequel le ou les condensats ou leur partie soumise à la rectification à basse température est ou sont en outre refroidis à un niveau de température allant de -30 à -30 °C en transférant de la chaleur à la première fraction.

10. Procédé selon la revendication 6, dans lequel le ou les condensats ou leur partie soumise à la rectification à basse température est ou sont en outre refroidis à un niveau de température allant de -40 à -100 °C en transférant de la chaleur à la première fraction et à un fluide frigorigène.

11. Procédé selon la revendication 9 ou 10, dans lequel la première fraction est détendue avant que la chaleur provenant du ou des condensats ou de leur partie soumise à une rectification redressement à basse température lui soit transférée.

12. Procédé selon la revendication 11, dans lequel la première fraction est amenée après le transfert de chaleur à l'adsorption modulée en pression (7).

13. Procédé selon l'une quelconque des revendications 4 à 12, dans lequel une partie de l'insert de séparation restant gazeux lors de la formation des condensats est aussi amenée au moins partiellement à la rectification à basse température.
